# EUROPEAN PATENT SPECIFICATION

(11) **EP 3 085 340 B1**
(45) Date of publication and mention of the grant of the patent: **14.04.2021**
(21) Application number: 14872805.8
(22) Date of filing: 16.12.2014
(51) Int. Cl.: A61F 2/966, A61F 2/962, A61F 2/954, A61F 2/95

(54) **STENT DELIVERY SYSTEM AND POST RELEASE ASSEMBLY THEREOF**
STENTEINFÜHRUNGSSYSTEM UND ANORDNUNG ZUR FREISETZUNG DANACH
SYSTÈME DE POSE D'ENDOPROTHÈSE ET SON ENSEMBLE POST-LIBÉRATION

(30) Priority: 17.12.2013 CN 201310693077
(43) Date of publication of application: 26.10.2016
(73) Proprietor: Shanghai MicroPort Endovascular MedTech (Group) Co., Ltd., Shanghai 201318 (CN)
(72) Inventor: LI, Zhonghua, Shanghai 201318 (CN); ZHU, Qing, Shanghai 201318 (CN); ZHU, Yongfeng, Shanghai 201318 (CN); LUO, Qiyi, Shanghai 201318 (CN); HUANG, Dingguo, Shanghai 201318 (CN); JI, Qingru, Shanghai 201318 (CN); LI, Biao, Shanghai 201318 (CN)
(74) Representative: Patentship Patentanwaltsgesellschaft mbH
(86) International application number: PCT/CN2014/093973
(87) International publication number: WO 2015/090188

(56) References cited:
- WO-A1-2011/163386
- CN-A- 102 038 565
- CN-A- 102 958 474
- CN-A- 103 230 310
- CN-U- 203 619 729
- US-A1- 2007 016 281
- US-A1- 2009 099 637
- US-A1- 2010 274 187
- US-A1- 2010 274 187
- US-A1- 2010 324 647
- US-A1- 2013 261 725

## Description

### TECHNICAL FIELD

This invention relates generally to the field of medical devices and, in particular, to an aortic stent delivery system used in interventional treatment of an aortic aneurysm (including an aortic dissection). The invention is also directed to a second release assembly of the delivery system.

### BACKGROUND

Aortic diseases such as aortic aneurysm and aortic dissection aneurysm are some of the deadliest and most intractable vascular surgical diseases. Traditionally, the treatment of such diseases relied on open surgery for artificial vessel replacement, which, however, led to significant trauma and high mortality.

In recent years, for high-risk aortic dissection patients, a simple, minimally-invasive interventional surgical procedure has been developed using a covered stent for endovascular repair of aortic dissection. In the procedure, the covered stent is introduced from a minimally invasive incision made in the patient's femoral artery and deployed to the aortic defect using a delivery system for protection and repair of the defective vessel.

Currently, delivery systems for use in minimally invasive intervention of aortas for the treatment of aortic aneurysm employ a simple covered stent deployment method involving retrieval of an outer sheath which allows the stent to expand by itself due to its elasticity. This method is, however, lack of accuracy in deployment control, because once the stent is improperly located, its self-expansion immediately after the retrieval of the outer sheath will necessitate the use of the highly traumatic open surgery with high fatality for removing the stent.

Chinese Utility Model Patent No. CN202699194U discloses a bifurcated abdominal aortic covered stent and a related delivery system. The bifurcated abdominal aortic covered stent includes a tubular lattice frame of a shape memory alloy and a cover covering the tubular lattice frame, in which at least two sleeve branches project from a leading portion of the tubular lattice frame. The delivery system includes a deployment assembly that is extendable within the tubular lattice frame and comprised of guide wires and flexible sleeves. Each flexible sleeve has a leading portion that can extend through the tubular lattice frame into a corresponding one of the sleeve branches in a shape-adapted manner, and each guide wire has one end arranged at a trailing end of a corresponding flexible sleeve and the other end disposed within the flexible sleeve and in connection with the corresponding sleeve branch. However, this delivery system is still not accurate enough in deployment control for the covered stent, and thus makes it necessary to rely on open surgery for removal of the covered stent when it is deployed at an undesired location.

International Pub. No. WO2011163386A1 discloses mechanisms for pulling a tensile member a predetermined distance from a medical device having an intracorporeal end and an extracorporeal end. Such mechanisms may be safely operated using a robot, two hands, or in some embodiments, only one hand. Such mechanisms may include one or more cams, drums, or pulley-like members and a lever, and may be physically coupled to an extracorporeal portion of the medical device.

U.S. Patent Pub. No. US2010274187A1 discloses a stent graft which is deployed by a steerable catheter delivery system having an integral tip capture release mechanism. The steering mechanism provides for a locked interference with a distal lock at the distal end of the delivery catheter. The configuration allows for selective circumferentially distributed release of one half or less of the number of crowns of a proximal spring which are captured by a tip capture mechanism so that new positioning of the stent graft can be verified and assured before full release of all proximal spring crowns is done. In this way, one or more steering elements of a catheter can be maintained in tension until catheter position is verified and acceptable stent graft position is achieved. This apparatus and method is particularly useful for deploying stent graft in curved passages such a thoracic aorta.

U.S. Patent Pub. No. US2007016281A1 discloses an introducer apparatus for deploying a self-expandable medical device, such as a stent, to a target area of a body vessel of a patient, the introducer apparatus including a shaft having a proximal end and a distal end, and a distal end portion disposed at the shaft distal end. The distal end portion includes an introducer body and at least one deployment member. The introducer body is sized and shaped relative to the self-expandable medical device such that the medical device is receivable on a surface of the introducer body when the medical device is in a compressed condition. The deployment member is configured and arranged relative to the introducer body for selectively restraining the self-expandable medical device in the compressed condition on the introducer apparatus surface.

### SUMMARY OF THE INVENTION

The present invention addresses the inaccurate deployment control issue of the current delivery systems for use in minimally invasive intervention of aortas for the treatment of aortic aneurysm by presenting a delivery system which allows second release of a proximal end of the stent, i.e., the stent being incompletely deployed with its proximal end being still restrained even after the outer sheath has been retrieved. In such a configuration, fine adjustments can still be made in the location of the stent to make sure that the stent is accurately deployed.

In one aspect, the present invention provides a second release assembly for use in a delivery system. The second release assembly is disposed at a proximal end of the delivery system and includes, sequentially from a proximal end to a distal end, a proximal fixing part, a restrain part and a control guidewire. A proximal end of the restrain part is detachably connected to the proximal fixing part, and a distal end of the restrain part is coupled to the control guidewire, wherein: the restrain part includes a section defining a bore allowing an inner tube of the delivery system to pass therethrough; the section has two additional holes formed therein and the two additional holes are configured for two strands of the control guidewire to extend therethrough; the restrain part further comprises a plurality of rods which are circumferentially distributed; the section is connected to or integrally formed with the plurality of rods. With this design, the stent can be incompletely deployed, i.e., the main part of the stent having expanded with its proximal end still being restrained. In this configuration, fine adjustments are still possible in the position of the stent. As a result, the proximal end can be deployed, resulting in complete deployment of the stent, after the stent has been tuned to a desired position.

According to the present invention, the restrain part may be a single part or a combination of several parts. The restrain part may be formed of a material which is a polymer or a metal.

Preferably, the second release assembly further includes a guide part, the restrain part is configured to extend through the guide part and is thus directed and maintained at a predetermined orientation by the guide part. The guide part may also be formed of a material which is a polymer or a metal.

According to an embodiment of the present invention, the proximal fixing part is provided with a plurality of blind holes which are circumferentially distributed and correspond to the plurality of rods, and each of the plurality of rods is inserted in a corresponding one of the plurality of blind holes.

According to an embodiment of the present invention, the guide part is provided with a plurality of guide holes which are circumferentially distributed and correspond to the plurality of rods, and wherein each of the plurality of rods extends through a corresponding one of the plurality of guide holes. With this design, the guide part can direct each leg of the restrain part to a desired orientation and maintain it thereat without intersecting or bending. This also facilitates alignment and insertion of the rods of the restrain part into the respective blind holes of the proximal fixing part.

In a second aspect, the present invention provides a stent delivery system including an outer sheath, an inner tube and a stent. The delivery system further includes a second release assembly as defined above to control the deployment of a proximal end of the stent. The second release assembly extends longitudinally within the outer sheath and receives the inner tube. The stent is provided with through holes at the proximal end, and the restrain part of the second release assembly extends through the through holes, thereby restraining the proximal end of the stent. With this design, the second release assembly can work together with the stent that is provided with the through holes at the proximal end, to restrain the proximal end of the stent by the second release assembly. As such, after the outer sheath has been retrieved, the stent will expand in the most part, with its proximal end still being restrained to an unexpanded configuration. At this time, the stent may be finely adjusted in its position such that it is accurately positioned at a desired site. After it is confirmed that the stent has been accurately located, the second release assembly may be controlled to deploy the proximal end of the stent, thereby resulting in complete expansion thereof.

Preferably, the delivery system further includes a conical tip disposed at a proximal end of the delivery system and is coupled to the proximal end of the proximal fixing part.

According to the present invention, the delivery system and the second release assembly enable delayed and, hence, more accurate deployment of the stent.

### BRIEF DESCRIPTION OF THE DRAWINGS

The features and advantages of the present invention will become readily apparent from the following detailed description of a few embodiments thereof, which is to be read in connection with the accompanying drawings.
Fig. 1 is a schematic perspective view of a second release assembly in accordance with an embodiment of the present invention;
Fig. 2 is a schematic perspective view of a proximal fixing part of a second release assembly in accordance with an embodiment of the present invention;
Fig. 3 is a schematic perspective view of a guide part of a second release assembly in accordance with an embodiment of the present invention;
Fig. 4 is a schematic perspective view of a restrain part of a second release assembly in accordance with an embodiment of the present invention;
Fig. 5 is a schematic perspective view of a restrain part of a second release assembly in accordance with another embodiment of the present invention;
Fig. 6 is a schematic perspective view of a restrain part of a second release assembly in accordance with yet another embodiment of the present invention;
Fig. 7 is a schematic illustrating a proximal end of a stent according to the present invention; and
Figs. 8 to 10 schematically illustrate a process of deploying a stent from a delivery system in accordance with an embodiment of the present invention.

### DETAILED DESCRIPTION

Embodiments of the inventive aortic stent delivery system will be described below with reference to the accompanying drawings. In the following description, for the sake of convenience, the terms "distal", "proximal" and "longitudinal" are used, in which "distal" and "proximal" refer to positions located close to and far away from a manipulated end of the delivery system, respectively, and "longitudinal" refers to a direction in which the delivery system extends.

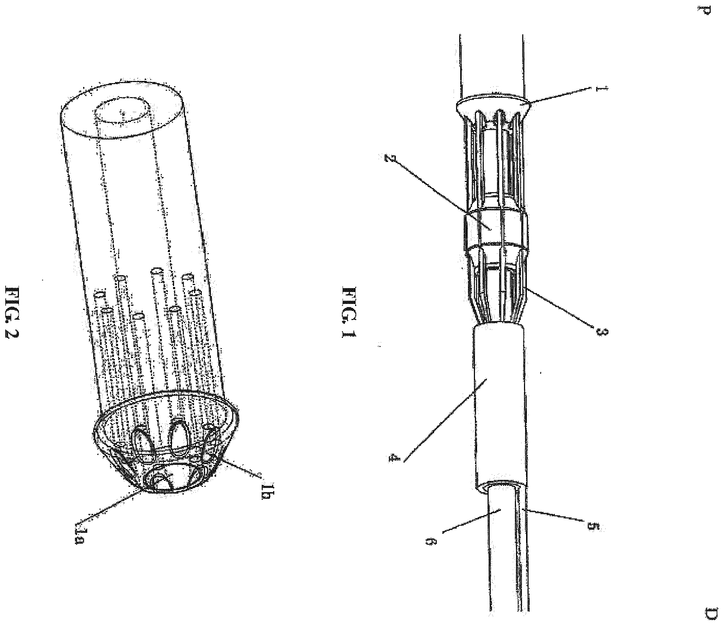

FIG. 1 shows a second release assembly in accordance with an embodiment of the present invention. As shown in Fig. 1, the second release assembly includes, sequentially from a proximal end P to a distal end D, a proximal fixing part 1, a guide part 2, a restrain part 3, a coupler 4 and a control guidewire 5. Fig. 1 also shows an inner tube 6 of the delivery system which extends through each of these components of the second release assembly.

Fig. 2 is a diagram showing the proximal fixing part 1. As shown in Fig. 2, the proximal fixing part 1 has an aperture 1a for the inner tube 6 to pass through, as well as a number of circumferentially distributed blind holes 1b. The number of the blind holes corresponds to the number of rods 3a of the restrain part 3 so that the rods 3a of the restrain part 3 can extend through the respective blind holes 1b. The proximal fixing part 1 may be injection molded, machined or fabricated by another technique.

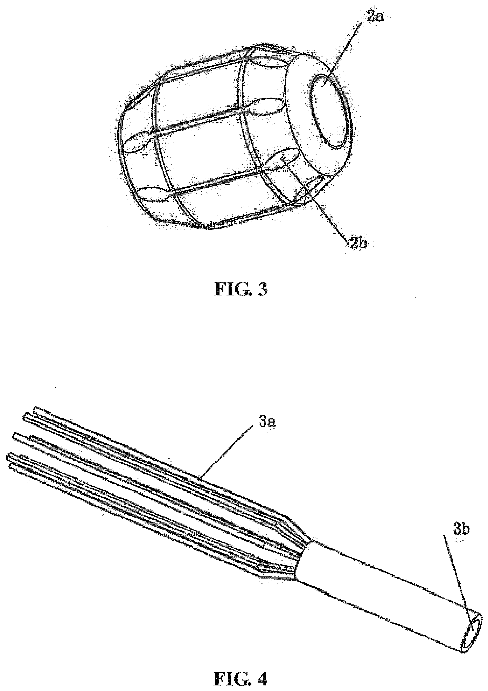

Fig. 3 is a diagram showing the guide part 2. As shown in Fig. 3, the guide part 2 has an aperture 2a for the inner tube 6 to pass through, as well as a number of circumferentially distributed guide holes 2b. The number of the guide holes corresponds to the number of rods 3a of the restrain part 3 so that the rods 3a of the restrain part 3 can extend through the respective guide holes 2b, thereby maintaining each of the rods 3a of the restrain part 3 in a desired orientation without intersecting or bending. This also facilitates alignment and insertion of the rods 3a of the restrain part 3 into the blind holes 1b of the proximal fixing part 1. The guide part 2 may be fabricated by injection molding, machining or another technique from a material which may be implemented as a polymer or a metal. The polymer herein may include, but not limited to, Pebax, Nylon, PC, PE, PP and ABS.

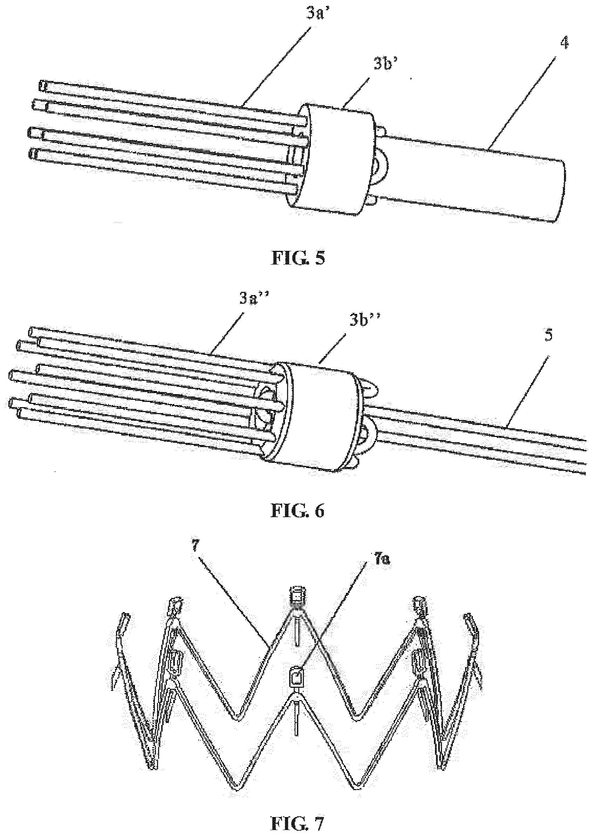

Figs. 4-6 are diagrams showing the restrain part 3. As shown in Fig. 4, the restrain part 3 includes a number of rods 3a which are circumferentially distributed and secured to a section 3b defining a bore for the inner tube 6 to pass through. The number of the rods depends on the number of through holes 7a (Fig. 7) provided at a proximal end of a stent 7. The rods 3a and the section 3b may be fabricated by injection molding, machining or another technique. The rods 3a and the section 3b may otherwise be connected to each other by, for example, but not limited to, welding, bonding or heat melting. Adhesives that can be used to effect the bonding include, but not limited to, Loctite 3011, 3321, 3493, 3494, 3321 and 3751; Dymax 203a-cth-f, 204-cth-f, 1201-m-sc and 1128a-m; NuSil MED-2000P; and Dow Corning Silastic Medical Adhesive Silicone, Type A. The material for fabricating the restrain part 3 may be a metal or a polymer such as, but not limited to, Pebax, Nylon, PC, PE, PP and ABS.

Of the restrain part 3, the rods 3a and the section 3b are formed as a single part (e.g., the case of the rods 3a' integral with the section 3b' shown in Fig. 5). Alternatively, the rods 3a and the section 3b are formed separately and then connected together (e.g. the case of the rods 3a" in fixed connection with the section 3b" shown in Fig. 6).

The coupler 4 is configured to couple the restrain part 3 to the control guidewire 5 to ensure that pulling the control guidewire 5 will lead to movement of the restrain part 3 toward the distal end.

The coupler 4 may be integrally formed with the section 3b', as shown in Fig. 5. Alternatively, the coupler 4 may be omitted, with the control guidewire 5 being directly coupled to the section 3b", as shown in Fig. 6. The coupling is accomplished by two additional holes in the section 3b" for two strands of the control guidewire 5 to extend therethrough. For ease of operation, a distal end of the double-stranded control guidewire 5 may be wrapped with a heat-shrink tube.

In the second release assembly, the rods 3a of the restrain part 3 that extend through the through holes 7a at the proximal end of the stent 7 of the delivery system and are inserted in the blind holes 1b of the proximal fixing part 1 restrain the proximal end of the stent 7. When to deploy the proximal end of the stent, the control guidewire 5 may be pulled to cause the restrain part 3 to be separated from the proximal fixing part 1 and move toward the distal end. Upon the rods 3a of the restrain part 3 leaving the through holes 7a of the stent 7, the proximal end of the stent 7 may expand.

A method for deploying the aortic stent 7 using the inventive delivery system may include the following steps.

In step 1, the stent 7 is loaded. The restrain part 3 is brought to move such that its rods are inserted through the guide holes 2b of the guide part 2. The proximal end of the stent 7 is crimped, and the restrain part 3 is further moved such that the rods 3a pass through the through holes 7a at the proximal end of the stent 7 and extend into the blind holes 1b of the proximal fixing part 1. As such, the proximal end of the stent 7 is restrained by the rods 3a and thus cannot expand.

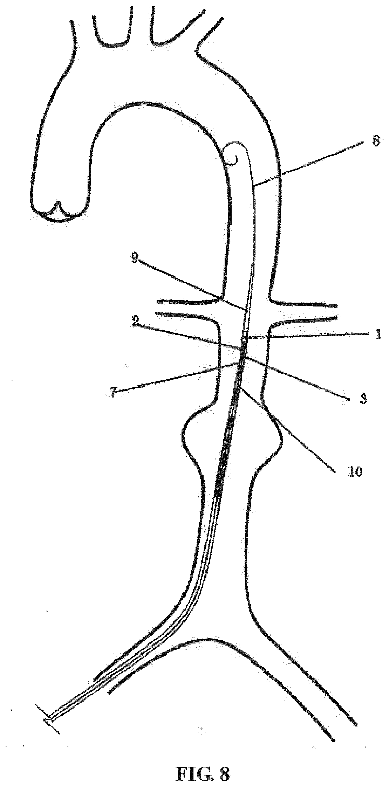

In step 2, the delivery system is introduced. As shown in Fig. 8, a guide wire 8 is first deployed in a path from a femoral artery to the abdominal aorta, and the delivery system incorporating the second release assembly on which the stent 7 is loaded is then percutaneously introduced and advanced over the guide wire 8 to the abdominal aorta. In the delivery system, the second release assembly extends longitudinally within an outer sheath 10, with the inner tube 6 extending through each of the above-described components of the second release assembly. In addition, the proximal fixing part 1 of the second release assembly is coupled to a conical tip 9.

In step 3, the delivery system is further advanced over the guide wire 8 to the site of an aortic aneurysm, with a radiopaque marker (not shown) being situated at a neck portion of the aneurysm, as shown in Fig. 8.

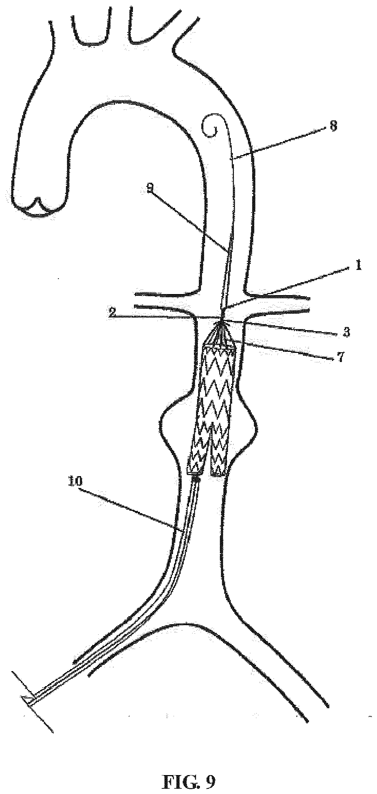

In step 4, the outer sheath 10 is retrieved. Specifically, after being properly located, the outer sheath 10 is retrieved to allow expansion of the trunk and branches of the stent 7 except the proximal end of the stent 7 which is still restrained by the second release assembly (Fig. 9).

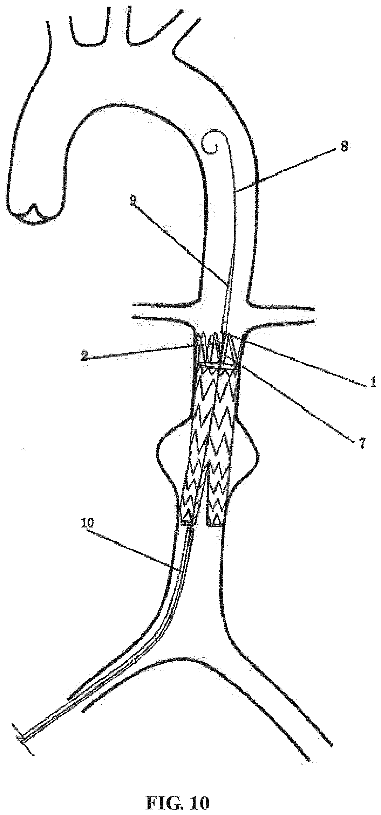

In step 5, the proximal end is deployed. Fine adjustments of the stent location are made if needed to make sure that the stent is located correctly. Thereafter, the control guidewire 5 is pulled to cause the restrain part 3 to move longitudinally toward the distal end. As a result, the rods 3a of the restrain part 3 are disengaged from the proximal fixing part 1 and then disengaged from the through holes 7a formed at the proximal end of the stent 7, thereby allowing expansion of the proximal end, i.e., complete deployment of the stent 7 (Fig. 10).

Finally, the delivery system and guide wire 8 are also retrieved, which lead to completion of the deployment of the stent 7.

## Claims

1. A second release assembly for use in a delivery system, the second release assembly being disposed at a proximal end of the delivery system, wherein the second release assembly comprises, sequentially from a proximal end to a distal end, a proximal fixing part (1), a restrain part (3) and a control guidewire (5), a proximal end of the restrain part (3) being detachably connected to the proximal fixing part (1), a distal end of the restrain part (3) coupled to the control guidewire (5);
the restrain part (3) comprises a section (3b, 3b', 3b") defining a bore allowing an inner tube (6) of the delivery system to pass therethrough; the restrain part (3) further comprises a plurality of rods (3a, 3a', 3a") which are circumferentially distributed; the section (3b, 3b', 3b") is connected to or integrally formed with the plurality of rods (3a, 3a', 3a"); **characterized in that**
the section (3b, 3b', 3b") has two additional holes formed therein and the two additional holes are configured for two strands of the control guidewire (5) to extend therethrough.

2. The second release assembly according to claim 1, further comprising a guide part (2), the restrain part (3) configured to extend through the guide part (2) and is thus directed and maintained at a predetermined orientation by the guide part (2).

3. The second release assembly according to claim 1, wherein the proximal fixing part (1) is provided with a plurality of blind holes (1b) which are circumferentially distributed and correspond to the plurality of rods (3a, 3a', 3a"), and wherein each of the plurality of rods (3a, 3a', 3a") is inserted in a corresponding one of the plurality of blind holes (1b).

4. The second release assembly according to claim 1, wherein the guide part (2) is provided with a plurality of guide holes (2b) which are circumferentially distributed and correspond to the plurality of rods (3a, 3a', 3a"), and wherein each of the plurality of rods (3a, 3a', 3a") extends through a corresponding one of the plurality of guide holes (2b).

5. A stent delivery system comprising an outer sheath (10), an inner tube (6) and a stent (7), wherein the delivery system further comprises a second release assembly as defined in any one of claims 1 to 4, the second release assembly being configured to control deployment of a proximal end of the stent (7), the second release assembly extending longitudinally within the outer sheath (10) and receiving the inner tube (6), and wherein the stent (7) is provided with through holes (7a) at the proximal end, and the restrain part (3) of the second release assembly extends through the through holes (7a), thereby restraining the proximal end of the stent (7).

6. The delivery system according to claim 5, wherein the delivery system further comprises a conical tip (9) disposed at a proximal end of the delivery system and coupled to the proximal end of the proximal fixing part (1).

## Patentansprüche

1. Anordnung zur zweiten Freisetzung zur Verwendung in einem Zuführungssystem, wobei die Anordnung zur zweiten Freisetzung an einem proximalen bzw. fernen Ende des Zuführungssystems angeordnet ist, wobei die zweite Freigabeanordnung aufeinanderfolgend von einem proximalen Ende zu einem distalen bzw. nahen Ende einen proximalen Fixierungsteil (1), einen Rückhalteteil (3) und einen Steuerführungsdraht (5) umfasst, wobei ein proximales Ende des Rückhalteteils (3) abnehmbar mit dem proximalen Fixierungsteil (1) verbunden ist, wobei ein distales Ende des Rückhalteteils (3) mit dem Steuerführungsdraht (5) gekoppelt ist;
wobei der Rückhalteteil (3) einen Abschnitt (3b, 3b', 3b") umfasst, der eine Bohrung definiert, durch die ein Innenrohr (6) des Zuführungssystems passieren kann; wobei der Rückhalteteil (3) ferner eine Vielzahl von Stäben (3a, 3a', 3a") umfasst, die umlaufend verteilt sind, wobei der Abschnitt (3b, 3b', 3b") mit der Vielzahl von Stäben (3a, 3a', 3a") verbunden ist oder mit der Vielzahl von Stäben (3a, 3a', 3a") einstückig ausgeformt ist;
**dadurch gekennzeichnet, dass**
der Abschnitt (3b, 3b', 3b") zwei in dem Abschnitt (3b, 3b', 3b") geformte zusätzliche Löcher aufweist und die zwei zusätzlichen Löcher dafür ausgebildet sind, dass sich zwei Stränge des Steuerführungsdrahts (5) durch sie hindurch erstrecken.

2. Anordnung zur zweiten Freisetzung nach Anspruch 1, ferner umfassend einen Führungsteil (2), wobei der Rückhalteteil (3) ausgebildet ist, sich durch den Führungsteil (2) hindurch zu erstrecken, und somit durch den Führungsteil (2) auf eine vorbestimmte Ausrichtung gerichtet und gehalten wird.

3. Anordnung zur zweiten Freisetzung nach Anspruch 1, wobei der proximale Fixierungsteil (1) mit mehreren Sacklöchern (1b) versehen ist, die umlaufend verteilt sind und der Vielzahl von Stäben (3a, 3a', 3a") entsprechen und wobei jeder der Vielzahl von Stäben (3a, 3a', 3a") in ein entsprechendes Sackloch der Vielzahl von Sacklöchern (1b) eingesetzt ist.

4. Anordnung zur zweiten Freisetzung, wobei der Führungsteil (2) mit einer Vielzahl von Führungslöchern (2b) versehen ist, die umlaufend verteilt sind und der Vielzahl von Stäben (3a, 3a', 3a") entsprechen, und wobei jeder der Vielzahl von Stäben (3a, 3a', 3a") sich durch ein entsprechendes Führungsloch der Vielzahl von Führungslöchern (2b) erstreckt.

5. Zuführungssystem für Stents bzw. endoluminale Gefäßprothesen, umfassend eine Außenhülle (10), ein Innenrohr (6) und einen Stent (7), wobei das Zuführungssystem ferner eine Anordnung zur zweiten Freisetzung gemäß einem der Ansprüche 1 bis 4 umfasst, wobei die Anordnung zur zweiten Freisetzung ausgebildet ist, den Einsatz eines proximalen Endes des Stents (7) zu steuern, wobei sich die Anordnung zur zweiten Freisetzung in Längsrichtung innerhalb der Außenhülle (10) erstreckt und das Innenrohr (6) aufnimmt, und wobei der Stent (7) am proximalen Ende mit Durchgangslöchern (7a) versehen ist und der Rückhalteteil (3) der Anordnung zur zweiten Freisetzung sich durch die Durchgangslöcher (7a) erstreckt, wodurch das proximale Ende des Stents (7) beschränkt wird.

6. Zuführungssystem nach Anspruch 5, wobei das Zuführungssystem ferner eine konische Spitze (9) umfasst, die an einem proximalen Ende des Zuführungssystems angeordnet ist und mit dem proximalen Ende des proximalen Fixierungsteils (1) gekoppelt ist.

## Revendications

1. Second ensemble de libération pour l'utilisation dans un système d'implantation, le second ensemble de libération étant disposé à une extrémité proximale du système d'implantation, le second ensemble de libération comprenant, successivement depuis une extrémité proximale jusqu'à une extrémité distale, une partie de fixation proximale (1), une partie de retenue (3) et un fil-guide de commande (5), une extrémité proximale de la partie de retenue (3) étant connectée de manière détachable à la partie de fixation proximale (1), une extrémité distale de la partie de retenue (3) étant accouplée au fil-guide de commande (5) ;
la partie de retenue (3) comprenant une section (3b, 3b', 3b") définissant un alésage permettant le passage d'un tube intérieur (6) du système d'implantation ;
la partie de retenue (3) comprenant en outre une pluralité de tiges (3a, 3a', 3a") qui sont réparties circonférentiellement ; la section (3b, 3b', 3b") étant connectée à, ou formée intégralement avec, la pluralité de tiges (3a, 3a', 3a") ;
**caractérisé en ce que**
la section (3b, 3b', 3b") présente deux trous supplémentaires formés dans celle-ci et les deux trous supplémentaires sont configurés pour permettre le passage à travers eux de deux brins du fil-guide de commande (5).

2. Second ensemble de libération selon la revendication 1, comprenant en outre une partie de guidage (2), la partie de retenue (3) étant configurée pour s'étendre à travers la partie de guidage (2) et étant ainsi dirigée et maintenue suivant une orientation prédéterminée par la partie de guidage (2).

3. Second ensemble de libération selon la revendication 1, dans lequel la partie de fixation proximale (1) est pourvue d'une pluralité de trous borgnes (1b) qui sont répartis circonférentiellement et qui correspondent à la pluralité de tiges (3a, 3a', 3a"), et dans lequel chacune parmi la pluralité de tiges (3a, 3a', 3a") est insérée dans un trou borgne correspondant parmi la pluralité de trous borgnes (1b).

4. Second ensemble de libération selon la revendication 1, dans lequel la partie de guidage (2) est pourvue d'une pluralité de trous de guidage (2b) qui sont répartis circonférentiellement et qui correspondent à la pluralité de tiges (3a, 3a', 3a"), dans lequel chacune parmi la pluralité de tiges (3a, 3a', 3a") s'étend à travers un trou de guidage correspondant parmi la pluralité de trous de guidage (2b).

5. Système d'implantation d'endoprothèse comprenant une gaine extérieure (10), un tube intérieur (6) et une endoprothèse (7), le système d'implantation comprenant en outre un second ensemble de libération selon l'une quelconque des revendications 1 à 4, le second ensemble de libération étant configuré pour commander le déploiement d'une extrémité proximale de l'endoprothèse (7), le second ensemble de libération s'étendant longitudinalement à l'intérieur de la gaine extérieure (10) et recevant le tube intérieur (6), et l'endoprothèse (7) étant pourvue de trous traversants (7a) au niveau de l'extrémité proximale, et la partie de retenue (3) du second ensemble de libération s'étendant à travers les trous traversants (7a), pour ainsi retenir l'extrémité proximale de l'endoprothèse (7).

6. Système d'implantation selon la revendication 5, le système d'implantation comprenant en outre une pointe conique (9) disposée à une extrémité proximale du système d'implantation et accouplée à l'extrémité proximale de la partie de fixation proximale (1).
